# EUROPEAN PATENT APPLICATION

(11) **EP 4 242 296 A1**
(43) Date of publication of application: **13.09.2023**
(21) Application number: 20960325.7
(22) Date of filing: 05.11.2020
(51) Int. Cl.: C12N 1/21, C12N 15/113, C12N 15/74, A61K 39/112, A61K 48/00, A61P 35/00, C12R 1/42

(54) **METHOD FOR CONSTRUCTING STRICTLY ANAEROBIC SALMONELLA, CONSTRUCTED STRICTLY ANAEROBIC SALMONELLA AND APPLICATION THEREOF**

(71) Applicant: Shenzhen Institutes of Advanced Technology Chinese Academy of Sciences, Shenzhen, Guangdong 518055 (CN)
(72) Inventor: LIU, Chenli, Shenzhen, Guangdong 518055 (CN); WANG, Zuowei, Shenzhen, Guangdong 518055 (CN); SHENG, Fangqian, Shenzhen, Guangdong 518055 (CN); ZENG, Zhengyang, Shenzhen, Guangdong 518055 (CN); LU, Weiqi, Shenzhen, Guangdong 518055 (CN); GUO, Xuan, Shenzhen, Guangdong 518055 (CN)
(74) Representative: Michalski Hüttermann & Partner Patentanwälte mbB
(86) International application number: PCT/CN2020/126769
(87) International publication number: WO 2022/094864

(57) **Abstract**

The invention relates to a method for constructing an obligate anaerobic *Salmonella* strain, the obligate anaerobic *Salmonella* strain constructed by using said method, and the use thereof in tumor therapy.

## Description

### Technical Field

The invention relates to the field of tumor targeted therapy, and in particular, to a method for constructing obligate anaerobic *Salmonella* strains, the obligate anaerobic *Salmonella* strains constructed by using the method and use thereof in tumor therapy.

### Background of the Invention

Cancer is the leading cause of death worldwide. Compared with normal cells, cancer cells are characterized as infinitely proliferating, transformable and easily metastasized. In addition to uncontrollably dividing (capable of multipolar division), cancer cells can also locally invade into the surrounding normal tissues and even metastasize to other organs through the body's circulatory or lymphatic systems. The history of cancer therapy shows that traditional methods such as surgery, chemotherapy, radiation therapy, immunotherapy, hormone therapy, bone marrow/stem cell transplantation, etc. have certain defects. For example, the problem of surgery therapy is that there is a risk of recurrence and some tumors are not easy to remove, and that of chemotherapy is it can cause serious side effects on patients and lead to ineffective treatment. The difficulties in cancer treatment stem from its complex and variable etiology. Not only are there changes in the genetic level of the body, but changes in the external environment are also one of the important factors for the development of cancer. The disadvantage of traditional therapies such as long-term radiation therapy, chemotherapy and immunotherapy is that these treatment options not only produce seriously toxicity to normal tissues and organs, but also cause cancer cells to develop multiple drug resistance and fail to completely kill cancer cells. In recent years, several studies have found that gene therapy, non-invasive radiofrequency cancer treatment, insulin boosting therapy, dietary therapy and bacterial therapy can not only prevent cancer cells from developing multi-drug resistance, but also enhance the efficacy of traditional therapies. Among them, bacterial therapy is a promising cancer treatment to overcome the shortcomings of conventional treatments.

The utilization of live bacteria to treat cancer dates back to more than 150 years ago. In 1868, German physician W. Bush used bacteria to treat an inoperable sarcoma, and within a week of treatment, the patient's neck lymph nodes and tumor had reduced in size. In 1883, the German surgeon Friedrich Fehleisen identified *Streptococcus pseudomallei* as the cause of septicemia. Subsequently, Friedrich Fehleisen and Willian B Coley, a surgeon from the New York Hospital, independently conducted experiments showing that *Streptococcus pseudomallei* could lead tumors of patients to regress. However, the results were controversial because the experimental results were difficult to repeat and did not meet the clinical criteria at that time. In 1935, Connell observed that enzymatic filtrates from *Clostridium perfringens* could cause metastases to regress. In 1947, Scientists injected spores of *Clostridium histolyticum* into mice transplanted with sarcomas for the first time and observed lysis of cancer cells and regression of tumor tissue. However, the percent survival of the mice was low due to the acute toxic reaction caused by the bacteria. In 1959 BCG (attenuated *Mycobacterium bovis*) was successfully used for cancer immunotherapy. In 2002, Phase I clinical trials with attenuated Salmonella VNP20009 (msbB-, purI-) showed that this strain could colonize in tumor tissue, but it was not effective for tumor therapy.

Although VNP20009 did not yield good clinical results, given the tumor-aggregating growth and immunomodulatory functions of Salmonella, scientists believe that multiple modifications might make Salmonella suitable for oncology treatment. The reason why we need to modify wild-type Salmonella is due to its virulence, which can cause fever, vomiting, diarrhea and abdominal cramps, and life-threatening bacteremia. With the rapid development of molecular biology technologies, Salmonella can be modified through different strategies to make it suitable for tumor therapy. It is possible to delete the virulence-related genes of Salmonella, to construct nutrient-deficient strains or to regulate bacterial growth through genetic circuits. To make the attenuated strains apply to clinical treatment as soon as possible.

Bin Yu et al. 2012, a research paper in the journal SCIENTIFIC REPORTS entitled "Explicit hypoxia targeting with tumor suppression by creating an 'obligate' anaerobic Salmonella Typhimurium strain", disclosed a protocol for constructing a strictly anaerobic Salmonella strain. Bin Yu *et al.* constructed a Salmonella typhimurium SL7207 strain by deleting the essential gene of asd. The deletion of asd gene affects bacterial cell wall production, and the addition of DAP (diaminoheptanedioic acid), a downstream intermediate metabolite of asd gene, in LB medium can enable normal cell wall synthesis. An anaerobic strain YB1 was then constructed by inserting the Cm-pept-asd-sodA anaerobically regulated gene circuit into the genome of SL7207 asd-deleted strain (the gene circuit was inserted into the original asd gene position). Fnr is a transcriptional regulator regulated by oxygen. Under anaerobic conditions, FNR is activated to regulate the forward promoter Pept to enable transcription of the asd gene so that the bacteria can produce an intact cell wall. The reverse promoter PsodA can block the leakage of the forward promoter to produce the asd gene product under aerobic conditions. This design allows the YB 1 strain to grow only under anaerobic conditions, while DAP must be added to the medium to grow under aerobic conditions.

Bin Yu *et al.* characterized the ability of their YB1 strain to survive under different oxygen conditions. Under aerobic conditions, YB1 was unable to grow in LB (DAP-) medium and could grow in LB (DAP+) medium; under anaerobic conditions, YB 1 could grow in both LB (DAP+) and LB (DAP-) medium. Distribution and therapeutic effect of YB 1 strain in tumor-bearing mice were characterized. 26 days after the tail vein injection of YB1 strain in tumor-bearing mice, the strain was cleared from all normal tissues and organs, and bacteria were still present in tumor tissues (due to the low oxygen concentration and immunosuppressive environment of tumor tissues). Compared with the PBS group, the YB1 strain had the ability to inhibit tumor growth.

However, it requires up to 26 days for completely clearance of the YB 1 strain from normal tissues and organs, which was time-consuming and unsafe. Compared with the PBS group, there was a significant decrease (>5%) in body weight of mice after YB 1 was administered to the tail vein of tumor-bearing mice. As an important evaluation indicator of the health of mice, the significant decrease in body weight indicated that the bacterium had a strong toxicity on mice.

There is still a need in this field for a construction method to produce strains that can be readily cleared up in normal tissues and organs in a much shorter period of time, so as to alleviate the toxic side effects on the tumor-bearing mice due to the long-term retention of bacteria in the body, and thereby making the modified strains safer and more reliable and does not affect the effectiveness of the bacteria in treating tumors.

### Summary of the Invention

In order to solve the problems in the prior art, an object of the invention is to provide a method for constructing an obligate anaerobic *Salmonella* strain, the obligate anaerobic *Salmonella* strain constructed by using the method, and use thereof in tumor therapy.

In one aspect of the invention, provided is a method for turning a facultative anaerobic bacterium into an obligate anaerobic bacterium by means of a circuit of hypoxically- or strictly anaerobically-induced expression of an essential gene(s), wherein said obligate anaerobic bacterium, when used for tumor therapy, is capable of inhibiting tumor growth and reducing tumor volume.

In one aspect of the invention, in the method described above, the said facultative anaerobic bacterium comprises a bacterium of the family Enterobacteriaceae (*Escherichia coli, Klebsiella pneumoniae, Proteus, Enterobacter, Salmonella typhi, Salmonella, Shigella,* etc.), *Staphylococcus, Streptococcus, Pneumococcus, Bacillus anthracis,* and *Corynebacterium diphtheriae.*

In one further aspect of the invention, in the method described above, said facultative anaerobic bacterium is a strain of *Salmonella sp.*

In one further aspect of the invention, in the method described above, the strain of said facultative anaerobic *Salmonella* comprises a strain derived from human, chicken, dog, cattle, etc.

In one further aspect of the invention, in the method described above, said obligate anaerobic bacterium, when being cultured *in vitro* under aerobic conditions, requires further addition of 2,6-diaminoheptanedioic acid (2,6-Diaminopimelic acid) and an analogue thereof into the culture medium.

In one further aspect of the invention, in the method described above, said essential gene is *dapA* or *dapE,* and may further comprise one or more selected from the group consisting of *dapB, dapD, argD, dapF, murE, murF* or *lysA.*

In one further aspect of the invention, in the method described above, said strictly anaerobically regulatory gene circuit consists of an anaerobically activated promoter and the essential gene.

In one further aspect of the invention, in the method described above, said gene circuit of hypoxically- or strictly anaerobically-induced expression regulation of the essential gene is present in a chromosome or a plasmid vector.

In one further aspect of the invention, in the method described above, said anaerobically activated promoter is selected from the group consisting of Pept, Fnr-SP, Hip1, 1141018, Ptet-arcA, Ptet-Fnr, R1074, Ssbp1 and YsgAP.

In one aspect of the invention, in the above method, the transcription factor regulating the promoter is Fnr or arcA. The Fnr-SP promoter is regulated by the transcription factor Fnr and the Ptet-arcA promoter is regulated by the transcription factor arcA.

In one further aspect of the invention, in the method described above, said tumor and cancer comprise leukemia (chronic leukemia, acute leukemia), osteocarcinoma, lymphoma (non-Hodgkin's lymphoma, Hodgkin's lymphoma), intestinal cancer (colon cancer, rectal cancer), liver cancer, stomach cancer, pelvic cancer (cervical cancer, malignant ovarian tumor, endometrial cancer, ovarian cancer), lung cancer, breast cancer, pancreatic cancer, bladder cancer, prostate cancer, etc.

In one further aspect of the invention, provided is a method for treating cancer using a bacterium regulated by an anaerobic circuit, said bacterium comprising strictly hypoxic regulation of expression of an essential gene.

In one further aspect of the invention, said therapeutic method further comprises combined application with other therapy for treating cancer.

In one further aspect of the invention, in said therapeutic method, said bacterium is *Salmonella typhi.*

In one further aspect of the invention, in said therapeutic method, said facultative anaerobic bacterium is *Salmonella typhimurium.*

In one further aspect of the invention, in said therapeutic method, the strain of said facultative anaerobic *Salmonella* comprises those derived from human, chicken, dog, cattle, etc.

In one further aspect of the invention, in said therapeutic method, the facultative anaerobic bacterium comprises a bacterium of the family Enterobacteriaceae (*Escherichia coli, Klebsiella pneumoniae, Proteus, Enterobacter, Salmonella typhi, Salmonella, Shigella,* etc.), *Staphylococcus, Streptococcus, Pneumococcus, Bacillus anthracis* and *Corynebacterium diphtheriae,* etc.

In one further aspect of the invention, in said therapeutic method, said combined application with other therapy for treating cancer comprise: (a) bacteriotherapy of an anaerobic strain in combination with operative therapy; (b) bacteriotherapy of an anaerobic strain in combination with radiotherapy; (c) bacteriotherapy of an anaerobic strain in combination with a chemotherapeutic which comprises alkylating agents (nimustine, carmustine, lomustine, cyclophosphamide, isocyclophosphamide, glyfosfin, etc.), antimetabolites (furtulon, doxifluridine, 6-mercaptopurine, cytarabine, fluoroguanosine, tegafur, gemcitabine, carmofur, hydroxyurea, methotrexate, tegafur-uracil, ancitabine, etc.), antitumor antibiotics (actinomycin, aclarubicin, epirubicin, mitomycin, pelomycin, bleomycin, pirarubicin, etc.), phytogenic anticarcinogens (irinotecan, harringtonine, hydroxycamptothecin, vinorelbine, paclitaxel, taxotere, topotecan, vincristine, vindesine, vinblastine, etc.), hormones (atamestane, anastrozole, aminoglutethimide, letrozole, formestane, megestrol, tamoxifen, etc.), immunosuppressants and other anticancer medicaments such as asparaginase, carboplatin, cisplatin, Dacarbazine, Oxaliplatin, Eloxatin, Oxaliplatin, mitoxantrone, procarbazine, etc.; (d) bacteriotherapy of an anaerobic strain in combination with biotherapy; (e) bacteriotherapy of an anaerobic strain in combination with traditional Chinese medical herbal treatment.

In one further aspect of the invention, provided is a vector which is a prokaryotic cell comprising (a) a hypoxically- or strictly anaerobically-activated promoter; and (b) an essential gene under the regulation of the promoter in (a); wherein the promoter in (a) has a binding site to an anaerobically activated transcription factor.

In one further aspect of the invention, in the vector, said anaerobically activated promoter is selected from the group consisting of Pept, Fnr-SP, Hip1, I141018, Ptet-arcA, Ptet-Fnr, R1074, Ssbp1 and YsgAP.

In one further aspect of the invention, in the vector, said essential gene is *dapA* or *dapE*; and/or, said transcription factor is Fnr, arcA. The Fnr-SP promoter is regulated by the transcription factor Fnr; the Ptet-arcA promoter is regulated by the transcription factor arcA.

In one further aspect of the invention, in the vector, when being cultured *in vitro* under aerobic conditions, requires addition of 2,6-diaminoheptanedioic acid into the culture medium.

In one further aspect of the invention, provided is use of the obligate anaerobic *Salmonella* strain in expressing a medicament or as a vector carrying a medicament, said medicament being used for treating cancer.

In one further aspect of the invention, in said use, said medicament comprises: (a) expressing a protein substance or a polypeptide substance having a therapeutic effect on cancer; (b) expressing an RNA having a therapeutic effect on cancer; and (c) serving as a vector carrying a modified RNA medicament.

### Brief Description of the Drawings

Figure 1 shows the construction of strain R1074.
Figures 2A-2G are electrophoretic images of the construction of 9 strains (SL7207 (ΔdapA)-promoter-BBa_B0033-dapA).
Figures 3A, 3B and 3C show in vitro experiments of 9 strains. Figure 3A shows the photos of 9 strains (as shown by the strain abbreviation on the left side of each photo) incubated under aerobic conditions for 24-144 h, respectively. Figure 3B shows the photos of 9 strains (as shown by the strain abbreviation on the left side of each photo) incubated under anaerobic conditions for 24h, respectively. Figure 3C shows the measured OD600 values of 9 strains (as shown by the strain abbreviations on X-axis) incubated under anaerobic conditions for 24h.
Figures 4A- 4E show the in vivo experimental results of the 9 strains constructed.
Figures 5(A)-5(E) are electrophoretic images of the construction of 5 strains (SL7207 (ΔdapE)-promoter (R1074, YsgAP, Fnr-SP, Pept, Hip1)-BBa_B0033-dapE).
Figures 6A, 6B and 6C show in vitro experiments of 5 strains. Figure 6A shows the photos of 5 strains (as shown by the strain abbreviation on the left side of each photo) incubated in aerobic conditions for 24-72 h. Figure 6B shows the photos of 5 strains (as shown by the strain abbreviation on the left side of each photo) incubated in anaerobic conditions for 24 h, respectively. Figure 6C shows the measured OD600 values of 5 strains (as shown by the of strain abbreviation on the left side of each photo) incubated in anaerobic conditions for 24 h.
Figure 7 shows the in vivo experiment of strain SL7207 (ΔdapE)-R1074-BBa_B0033-dapE (abbreviation: R1074-1).

### Detailed Description of the Invention

Although various modifications can be made to the invention and there may be a variety of forms for the invention, the embodiments will be described and interpreted in detail as follows. However, it should be understood that these are not intended to limit the invention to a particular disclosure and that the invention comprises all modifications, equivalents or alternatives thereof without departing from the spirit and technical scope of the invention.

The method of constructing an obligate anaerobic *Salmonella* strain, the obligate anaerobic *Salmonella* strain constructed by using the method and the use thereof in tumor therapy according to specific embodiments of the invention will be interpreted in more detail hereinafter.

In one or more embodiments of the invention, the vector of the invention is a prokaryotic cell comprising (a) a hypoxically- or strictly anaerobically-activated promoter; and (b) an essential gene under the regulation of the promoter in (a), wherein the promoter in (a) has a binding site to an anaerobically activated transcription factor.

In one or more embodiments of the invention, the hypoxically or strictly anaerobically activated promoter in (a) may be, for example, Fnr-SP, Hip1, I14018, Ptet-arcA, Ptet-Fnr, R1074, Ssbp1 or YsgAP.

In one or more embodiments of the invention, said transcription factor is Fnr or arcA.

In one or more embodiments of the invention, said essential gene in (b) under the regulation of the promoter in (a) may be, for example, *dapA, dapB, dapD, argD, dapE, dapF, murE, murF, lysA,* etc.; in particular, *dapA* or *dapE.*

The invention provides a method for turning a facultative anaerobic bacterium into an obligate anaerobic bacterium by means of a circuit of hypoxically- or strictly anaerobically-induced expression of an essential gene.

In one or more embodiments of the invention, said strictly anaerobically regulatory gene circuit consists of an anaerobically activated promoter and the essential gene.

In one or more embodiments of the invention, the anaerobically activated promoter may be, for example, Pept, Fnr-SP, Hip1, I14018, Ptet-arcA, Ptet-Fnr, R1074, Ssbp1 or YsgAP.

In one or more embodiments of the invention, said essential gene may be, for example, *dapA, dapB, dapD, argD, dapE, dapF, murE, murF, lysA,* etc.; in particular, *dapA* or *dapE.*

When the essential gene is *dapA* or *dapE* gene, the vector of the invention, when being cultured under aerobic conditions, requires further addition of 2, 6-diaminoheptanedioic acid (2, 6-Diaminopimelic acid) or an analogue thereof into the culture medium.

The obligate anaerobic bacterium described in the invention, when used for *in vivo* tumor therapy, can inhibit tumor growth and reduce tumor volume.

Said facultative anaerobic bacterium may be any species from any one of the bacterial genera of bacteria of the family Enterobacteriaceae (*Escherichia coli, Klebsiella pneumoniae, Proteus, Enterobacter, Salmonella typhi, Salmonella, Shigella,* etc.), *Staphylococcus, Streptococcus, Pneumococcus, Bacillus anthracis* and *Corynebacterium diphtheriae* and the like.

The source of said facultative anaerobic *Salmonella* strains is not limited, as long as they are facultatively anaerobic, comprising, for example, facultative anaerobic *Salmonella* strains from human, chicken, dog, cattle, etc.

Said facultative anaerobic bacterium is *Salmonella typhimurium.*

The invention also provides a bacteriotherapy for cancer treatment by using the strains of the invention that are unable to grow under either aerobic or anaerobic conditions.

Said cancers comprise leukemia (chronic leukemia, acute leukemia), osteocarcinoma, lymphoma (non-Hodgkin's lymphoma, Hodgkin's lymphoma), intestinal cancer (colon cancer, rectal cancer), liver cancer, stomach cancer, pelvic cancer (cervical cancer, malignant ovarian tumor, endometrial cancer, ovarian cancer), lung cancer, breast cancer, pancreatic cancer, bladder cancer, prostate cancer, etc.

In one or more embodiments of the invention, the vector of the invention as a prokaryotic cell, or the obligate anaerobic bacterium obtained by the method of the invention, can be used as a bacteriotherapy for anti-tumor or cancer treatment.

In one or more embodiments of the invention, the bacteriotherapy of the invention can be used in combination with other therapy for treating cancer.

In one or more embodiments of the invention, the combined application of bacteriotherapy with other therapy for treating cancer comprises, for example, (a) bacteriotherapy of an anaerobic strain in combination with operative therapy; (b) bacteriotherapy of an anaerobic strain in combination with radiotherapy; (c) bacteriotherapy of an anaerobic strain in combination with chemical medicaments: chemotherapeutics comprising alkylating agents (nimustine, carmustine, lomustine, cyclophosphamide, isocyclophosphamide, glyfosfin, etc.), antimetabolites (furtulon, doxifluridine, 6-mercaptopurine, cytarabine, fluoroguanosine, tegafur, gemcitabine, carmofur, hydroxyurea, methotrexate, tegafur-uracil, ancitabine, etc.), antitumor antibiotics (actinomycin, aclarubicin, epirubicin, mitomycin, pelomycin, bleomycin, pirarubicin, etc.), phytogenic anticarcinogens (irinotecan, harringtonine, hydroxycamptothecin, vinorelbine, paclitaxel, taxotere, topotecan, vincristine, vindesine, vinblastine, etc.), hormones (atamestane, anastrozole, aminoglutethimide, letrozole, formestane, megestrol, tamoxifen, etc.), immunosuppressants and other anticancer medicaments such as asparaginase, Carboplatin, Cisplatin, Dacarbazine, Oxaliplatin, Eloxatin, Oxaliplatin, mitoxantrone, procarbazine, etc.; (d) bacteriotherapy of an anaerobic strain in combination with biotherapy; (e) bacteriotherapy of an anaerobic strain in combination with traditional Chinese medical herbal treatment.

The vector of the invention as a prokaryotic cell, or the obligate anaerobic bacterium obtained by the method of the invention, can also be used for inducing expression of a medicament *in vitro* or serving as a vector carrying a medicament, so as to carry out cancer treatment.

In an embodiment of the invention, the medicament that can be carried in said vector comprises: (a) expressing a protein substance or a polypeptide substance having a therapeutic effect on cancer; (b) expressing an RNA having a therapeutic effect on cancer; and (c) serving as a vector carrying a modified RNA medicament.

Advantages of the invention comprise that:
(1) The anaerobic regulatory module of the modified strain is simple, the regulatory system is more rigorous, and there is no intrinsic leaky expression under aerobic conditions;
(2) The modified strain can be completely cleared in normal tissues and organs within only a short time;
(3) During the treatment of tumor-bearing mice, the modified strain has almost no effect on the body weight of the mice, and the safety is improved with relatively small toxic side effects.

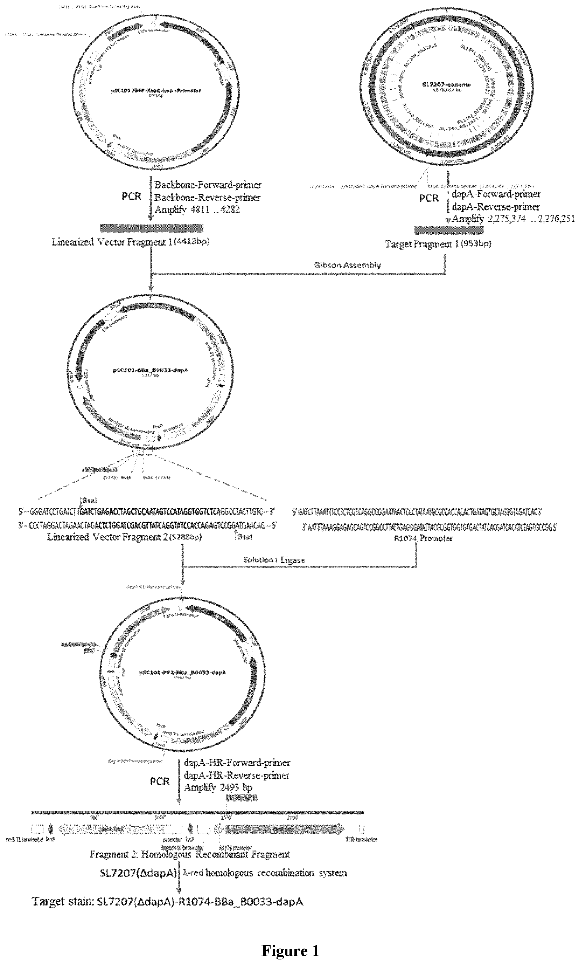

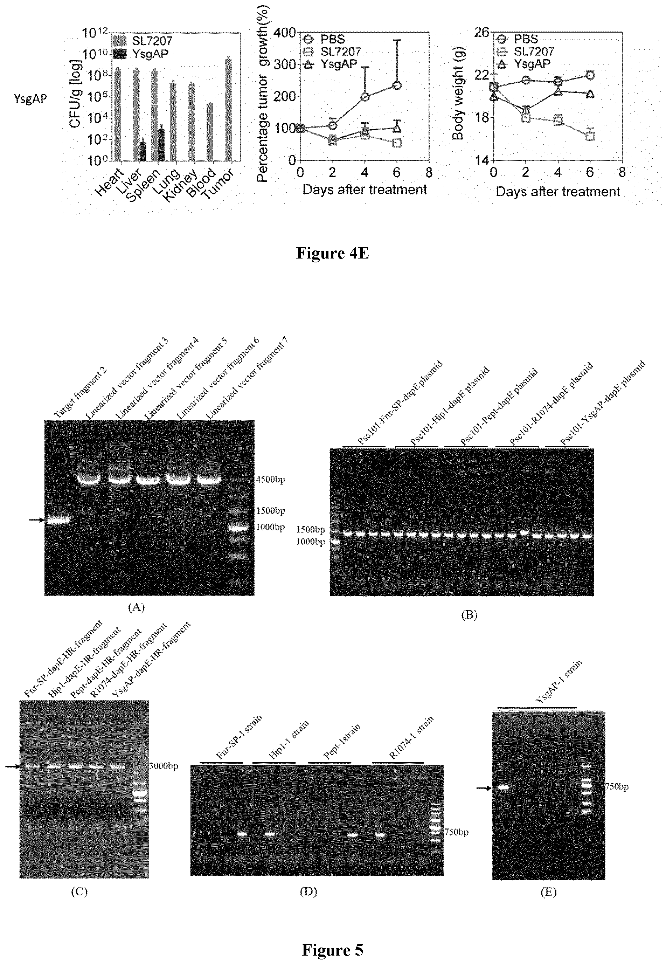

The strain construction scheme, as shown in Figures 1 and 5, comprises that: on the basis of SL7207 (ΔdapA) and SL7207 (ΔdapE) already constructed in our laboratory, Fnr-SP, Hip1, I14018, Pept, Ptet-arcA, Ptet-Fnr, R1074, Ssbp1 or YsgAP-BBa_B0033-dapA anaerobic gene circuit is integrated to SL7207 (ΔdapA) genome (original dapA position); and Fnr-SP, Hip1, Pept, R1074, YsgAP-BBa_B0033-dapE anaerobic gene circuit is integrated to SL7207 (ΔdapE) genome (original dapA position). The *dapA* and *dapE* genes are key genes in the lysine metabolism pathway, and knockout of either of these two genes prevents a bacterium from forming a normal cell wall, resulting in imbalance of the osmotic pressures inside and outside the bacterium, and prevents the bacterium from surviving due to cell rupture. The above promoters are anaerobically activated promoters, which can initiate the transcription of *dapA* or *dapE* gene under anaerobic or hypoxic conditions, thereby allowing the downstream DAP protein to generate DAP. The bacterium can form an intact cell wall. Under aerobic conditions, the 9 promoters-BBa_B0033-dapA or the 5 promoters-BBa_B0033-dapE gene circuits are inactivated and the bacteria are unable to produce an intact cell wall. Cultivation of SL7207 (ΔdapA)-Promoter (one of the 9 promoters as shown in Table 1)-BBa_B0033-dapA strain or SL7207 (ΔdapE)-Promoter (one of the 5 promoters)-BBa_B0033-dapE strain under aerobic conditions requires the addition of DAP (diaminoheptanedioic acid), which can compensate for the genetic deletion of *dapA* or *dapE* resulting in the inability of the bacteria to form an intact cell wall.

**Table 1. Promoters used in the invention**

| Promoter name | Sequence |
|---|---|
| Fnr-SP | 5′GATCCGCCGCAAAGTTTGAGCGAAGTCAATAAACTCTCTACCCATTCAGGG CAATATCTCTCTTGCAGGTGAATGCAACGTCAAGCGAT 3′ |
| Hip1 | 5′GATCGGATAAAAGTGACCTGACGCAATATTTGTCTTTTCTTGCTTAATAAT GTTGTCA 3′ |
| 114018 | 5' GATCTGTAAGTTTATACATAGGCGAGTACTCTGTTATGG 3' |
| Pept | 5′GATCGCAGGGGTAAAAGTGACCTGACGCAATATTTGTCTTTTCTTGCTTCT TAATAATGTTGTCACAAAAAGTGAGGGTGACTACATGG 3′ |
| Ptet-arcA | 5' GATCGTTAATAAAATGTTATTGACAGTTAATAAAATGTTATACTGAGC 3' |
| Ptet-Fnr | 5′GATCAAAATTGATCTGAATCAATATTTTGACAAAAATTGATCTGAATCAAT ATTTACTGAGC 3′ |
| R1074 | 5′GATCTTAAATTTCCTCTCGTCAGGCCGGAATAACTCCCTATAATGCGCCAC CACACTGATAGTGCTAGTGTAGATCAC 3′ |
| Ssbp1 | 5′GATCAACCGAGGTCACAACATAGTAAAAGCGCTATTGGTAATGGTACAATC GCGCGTTTACACTTATTCAGAACGATTTTTTTCAGGAG 3′ |
| YsgAP | 5′GATCTCAGAAGAAGCAAAAAGACACTTTACCGAAGGGTTTAACATTTTTTC GTGATACTCATCACCATGACGCAAATGCGTTGCATAAA 3′ |

**Table 2. Primers used for the cloning of the invention**

| Primer name | Sequence |
|---|---|
| Vector-Forward-primer 1 | 5'-gattacttcgcattgcaatcag-3' |
| Vector-Reverse-primer 2 | 5'-caagtaggcctgagaccac-3' |
| dapA-Forward-primer | 5'-gtggtctcaggcctacttgtcacacaggacggtac catgttcacgggaagtatt-3' |
| dapA-Reverse-primer | 5'-gattgcaatgcgaagtaatcttagtgatggtgatg gtgatgcagcaggccagcattg-3' |
| dapA-HR-forward-primer | 5′-tgccataccaaacgtaccattgagggacttgtttg cacagaggatggccccgaagaaaggcccacccgtg-3′ |
| dapA-HR-Reverse-primer | 5′-tcgccaggcgcgacttttgtactgagtaagccatc aaatctccctaaactatttgtcctactcaggagagcgt tc-3′ |
| dapA homologous recombination identification forward primer | 5'-ggaaaccataaaaaaaacctgcat-3' |
| dapA homologous recombination identification reverse primer | 5'-aacgtagcggcgcgagat-3' |
| Vector-Forward-primer 3 | 5'-gattacttcgcattgcaatcag-3' |
| Vector-Reverse primer 4 | 5'-taccgtcctgtgtgacaagt-3' |
| dapE-Forward-primer | 5'-acttgtcacacaggacggatgtcgtgcccggttat tgag-3' |
| dapE-Reverse-primer | 5'-gattgcaatgcgaagtaatcttagtgatggtgatg gtgatgggcgacgagctgttccat-3' |
| dapE-HR-Forward-primer | 5′-ttggtttcagtgaatcccgttatcagcagtttttt gatgaggtgtagtctatttgtcctactcaggagagcgt tc-3′ |
| dapE-HR-Reverse-primer | 5'-caatattttgccagccagtccatgcttatttcctc ttaccggaacgctcacgaagaaaggcccacccgtg-3' |

### Examples

### Example 1: Construction of 9 obligate anaerobic strains (SL7207 (AdapA)-Promoters-BBa_B0033-dapA) (primers used in the following experiments are shown in Table 2)

1. Construction of pSC101-BBa_B0033-dapA plasmid
   a. Linearized vector fragment 1 was obtained via PCR using pSC101-FbFp-KnaR-loxp + promoter plasmid as the template, with Backbone-Forward-primer and Backbone-Reverse-primer as primers;
   b. Target fragment 1 was obtained via PCR using *Salmonella* SL7207 genome as the template, with dapA-Forward-primer and dapA-Reverse-primer as primers; and
   c. The pSC101-BBa_B0033-dapA plasmid was obtained via one-step cloning method.
2. Construction of pSC101-R1074-BBa_B0033-dapA plasmid
   a. The pSC101-BBa_B0033-dapA plasmid was enzymatically cleaved by BsaI to obtain linearized vector fragment 2;
   b. Primer annealing method was carried out to obtain the R1074 promoter fragment;
   c. The pSC101-R1074-BBa _B0033-dapA plasmid was obtained by ligating by ligase.
3. Construction of SL7207 (ΔdapA)-R1074-BBa_B0033-dapA strain
   a. Homologous recombinant (HR) fragment 2 was obtained via PCR using the pSC101-R1074-BBa_B0033-dapA plasmid as the template, with dapA-HR-Forward-primer and dapA-HR-Reverse primer as primers;
   b. The homologous recombinant fragment 2 was integrated to the original dapA gene position of SL7207 (ΔdapA) by λ-red homologous recombination method to obtain SL7207 (ΔdapA)-R1074-BBa_B0033-dapA target strain.

The other 8 target strains (below) were constructed in a manner essentially identical to the SL7207 (ΔdapA)-R1074-BBa_B0033-dapA strain, requiring the replacement of the b primer annealing fragment in step 2 with Hip1, I14018, Ptet-Fnr, PepT, Ptet-arcA, Ssbp1, Fnr-SP, and YsgAP promoter fragments.
1. SL7207 (ΔdapA)-Fnr-SP-BBa_B0033-dapA;
2. SL7207 (ΔdapA)-Hip1-BBa_B0033-dapA;
3. SL7207 (AdapA)-I14018-BBa_B0033-dapA;
4. SL7207 (ΔdapA)-Pept-BBa_B0033-dapA;
5. SL7207 (ΔdapA)-Ptet-arcA-BBa_B0033-dapA;
6. SL7207 (ΔdapA)-Ptet-Fnr-BBa_B0033-dapA;
7. SL7207 (ΔdapA)-Ssbp1-BBa_B0033-dapA;
8. SL7207 (ΔdapA)-YsgAP-BBa_B0033-dapA.

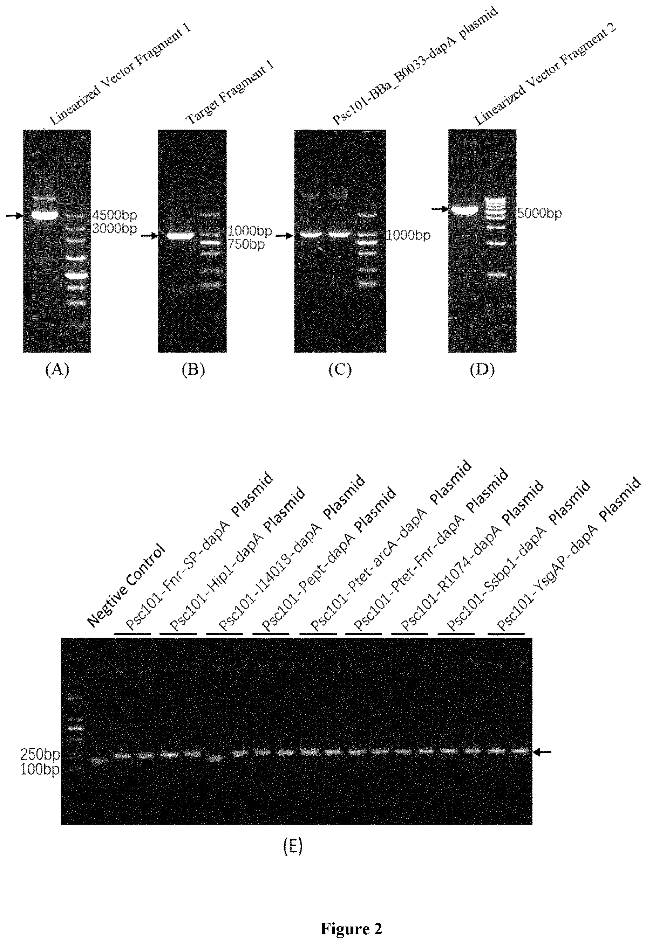

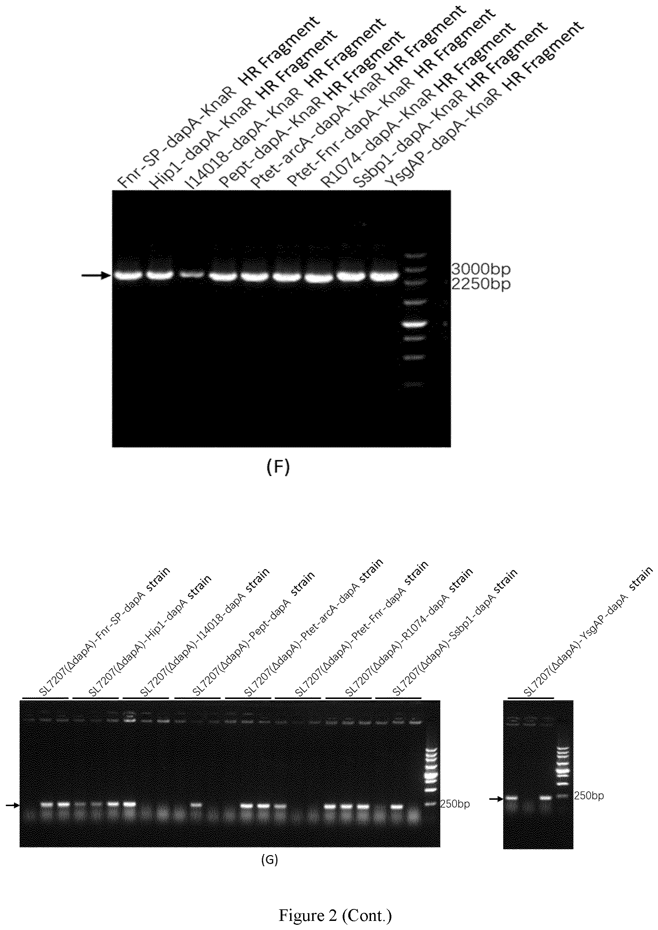

The 9 strains were abbreviated as (Fnr-SP; Hip1; I14018; Pept; Ptet-arcA; Ptet-Fnr; R1074; Ssbp1; YsgAP), and the electrophoresis results are shown in Figures 2A-2G.

### Example 2: In vitro characterization of the 9 strains

Characterization under aerobic conditions: 1 monoclone was picked and added into LB(DAP+) medium containing kanamycin; 3 monoclones were picked and added into LB(DAP-) medium containing kanamycin, respectively. The monoclones were incubated in an air bath shaker (37°C, 220 rpm) for a period of time.

Characterization under anaerobic conditions: 3 monoclones were picked and added into LB(DAP+) medium containing kanamycin. Overnight incubation was carried out in an air bath shaker (37°C, 220 rpm). The bacterial suspension after overnight incubation was placed in an anaerobic incubator and transferred at a ratio of 1:100. 20 µl of the bacterial suspension was taken and added into 2 ml of LB(DAP+) medium containing kanamycin; 20 µl of the bacterial suspension was taken and added into 2 ml of LB(DAP-) medium containing kanamycin, carried out in triplicate. The initial OD600 value of the samples after transferring was measured. The samples were incubated stationarily in an anaerobic incubator at 37°C for 24 h. After incubation for 24 h, the OD600 values of the samples were measured.

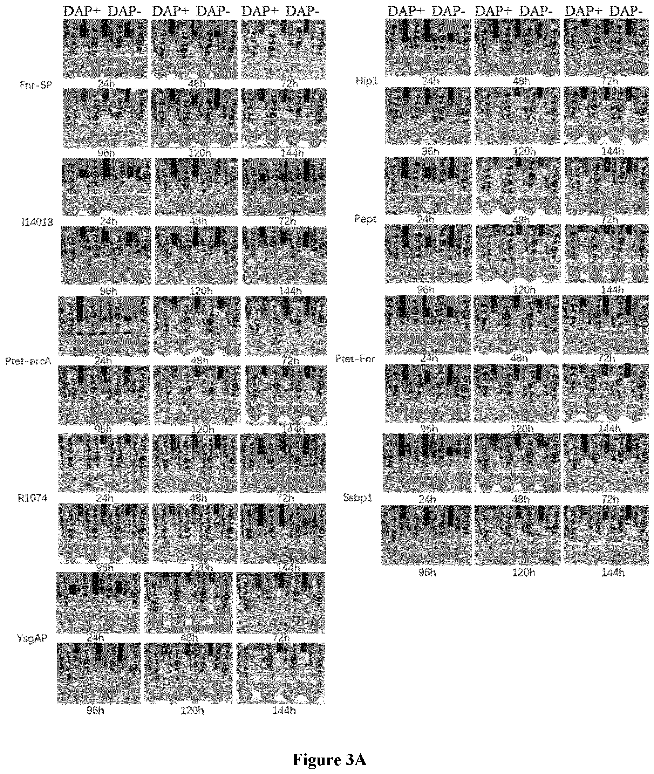

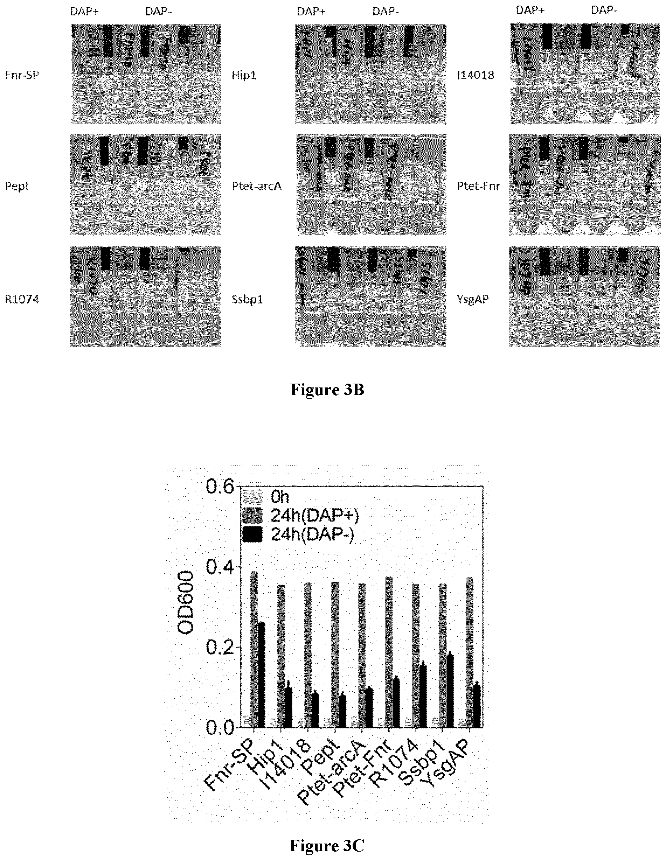

Experimental results (as shown in Figures 3A- 3C):
(1) Under aerobic condition: the 9 strains were incubated in LB(DAP+) medium for 144 h, and these strains could grow normally. The 9 strains were incubated in LB(DAP-) medium for 144 h, and these strains could not grow.
(2) Under anaerobic condition: the 9 strains were cultured in LB(DAP+) medium and in LB(DAP-) medium for 24h. These strains could grow in both LB(DAP+) medium and LB(DAP-) medium.

Conclusion: by means of the tests on the bacterial strains under aerobic and anaerobic conditions, it showed that the facultative anaerobic strain SL7207 was successfully modified into an obligate anaerobic strain.

### Example 3: In vivo characterization of the 9 strains

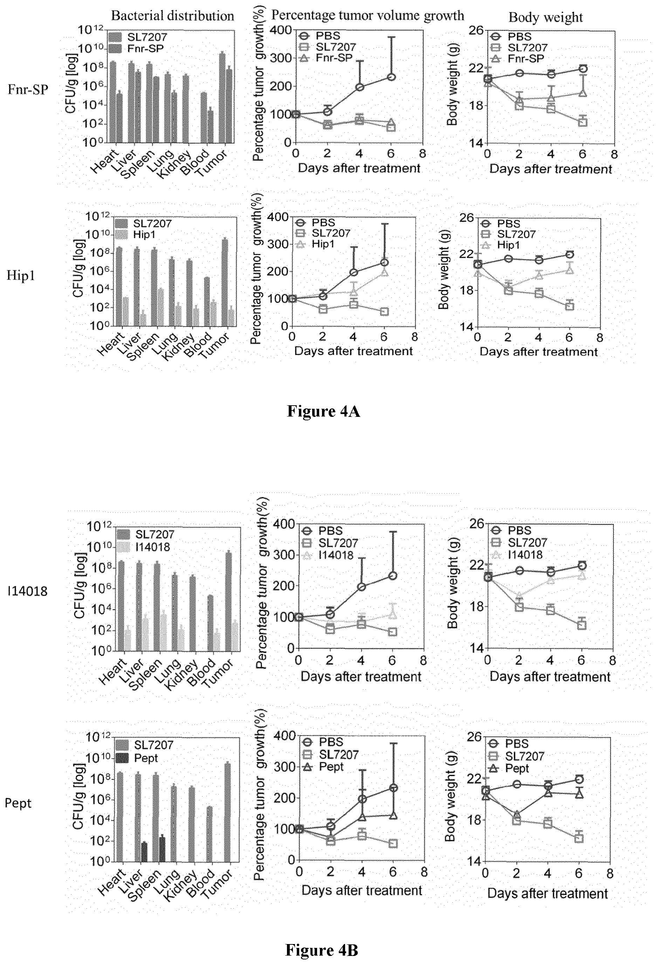

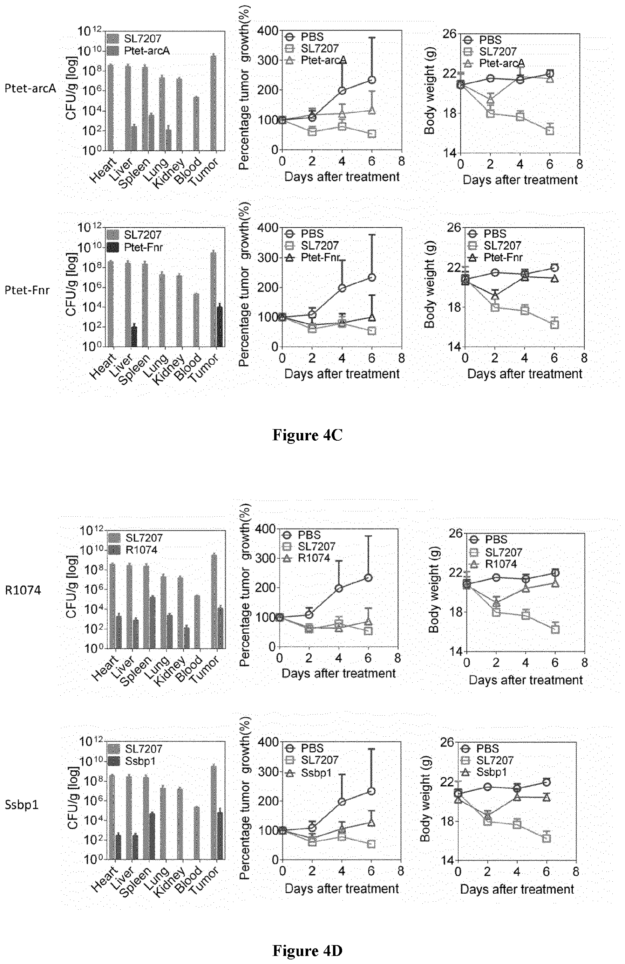

C57BL/6 mice were subcutaneously inoculated with 1×10⁶ mouse bladder cancer cells (MB49) per mouse to establish a subcutaneous tumor model of mouse bladder cancer. The experiment was divided into PBS group, SL7207 strain group, Fnr-SP group, Hip1 group, I14018 group, Pept group, Ptet-arcA group, Ptet-Fnr group, R1074 group, Ssbp1 group and YsgAP group. The tail vein was inoculated with 1×10⁷ of each type of bacteria of the invention per mouse. The distribution of each bacteria in normal tissues and organs and in tumors of tumor-bearing mice, the change of tumor volume, the change of mouse body weight, and the percent survival of mice were detected within 6 days. Experimental results comprise (as shown in Figures 4A- 4E):
(1) Distribution of bacteria in the tumor-bearing mice (Figures 4A-4E, left column): The bacterial strain of the Fnr-SP group was cleared slowly in vivo, and the normal and tumor tissues had a large number of the bacteria; the bacterial strains of the Hip1, I14018, R1074, and Ssbp1 groups were cleared relatively quickly in vivo. The amounts of the bacteria in heart, liver, lung, kidney and blood were relatively lower, and there were 10⁴ (CFU/g) bacteria in spleen and tumor tissues. The bacteria of the Pept and YsgAP groups were only present in small amounts in liver and spleen, and were absent in other tissues, organs and tumor. The bacteria of the Ptet-arcA group were only present in small amounts in heart, liver and lung, and were absent in spleen, kidney, blood and tumor tissues. The bacteria of the Ptet-Fnr group were only present in liver and tumor tissues, but were absent in other organs.
(2) Changes in tumor volume (Figures 4A-4E, middle column): compared with the PBS group, all the 9 strain groups had inhibitory effects on tumor growth within 6 days.
(3) Changes in body weight of mice (Figures 4A- 4E, right column): compared with SL7207 group, the mice of the 9 strain groups had less weight loss, which was slightly lower than that of PBS group.
(4) Percent survival of mice: all mice of SL7207 group died within 6 days. There were no deaths of mice in the 9 strain groups and PBS group during the experimental cycle.

Conclusion: within 6 days, the distribution of the 9 modified strains in normal tissues and organs in vivo was not identical to that in tumors. Compared with SL7207 group, the 9 strains of the invention were cleared in large amounts in vivo. The tumor volumes in 9 strain groups of the invention were all reduced. The mice of the 9 strain groups had slightly lower body weight than that of the PBS group during the experimental cycle, without any death. It indicates that the 9 strains of the invention have improved safety while having an inhibitory effect on tumors, compared with the prior art.

### Example 4: Construction of 5 obligate anaerobic strains (SL7207 (ΔdapE)-Promoter-BBa_B0033-dapE)

1. Construction of pSC101-Promoters-BBa_B0033-dapE plasmids
   a. Five linearized vector fragments were obtained via PCR by using pSC101-Promoters (R1074, YsgAP, Fnr-SP, Pept, Hip1)-BBa_B0033-dapA plasmids as the templates, with Backbone-Forward-primer 3 and Backbone-Reverse-primer 4 as primers (results as shown in Figure 5(A));
   b. Target fragment 2 was obtained via PCR by using *Salmonella* SL7207 genome as the template, with dapE-Forward-primer and dapE-Reverse-primer as primers (results as shown in Figure 5 (A));
   c. The pSC101-Promoters (R1074, YsgAP, Fnr-SP, Pept, Hip1)-BBa_B0033-dapE plasmids were obtained via one-step cloning method (results as shown in Figure 5 (B)).
3. Construction of SL7207 (ΔdapE)-Promoters (R1074, YsgAP, Fnr-SP, Pept, Hip1)-BBa_B0033-dapE strains
   a. Five homologous recombinant fragments were obtained via PCR by using pSC101-Promoters (R1074, YsgAP, Fnr-SP, Pept, Hip1)-BBa_B0033-dapE plasmids as the templates, with dapE-HR-Forward-primer and dapE-HR-Reverse-primer as primers (results are shown in Figure 5(C));
   b. The five homologous recombinant fragments were integrated into the original dapE gene position of SL7207 (ΔdapE) by λ-red homologous recombination method to obtain SL7207 (ΔdapE)-Promoters (R1074, YsgAP, Fnr-SP, Pept, Hip1)-BBa_B0033-dapE target strains (results as shown in Figures 5(D) and (E)).

The five strains were abbreviated as (R1074-1, YsgAP-1, Fnr-SP-1, Pept-1, Hip1-1).

### Example 5: In vitro characterization of the 5 obligate anaerobic strains (SL7207 (ΔdapE)-Promoters (R1074, YsgAP, Fnr-SP, Pept, Hip1)-BBa_B0033-dapE)

Characterization under aerobic conditions: 3 clones were picked and added into LB(DAP+) medium containing spectinomycin and incubated overnight in an air bath shaker (37°C, 220 rpm). The bacterial suspension after overnight incubation was transferred at a ratio of 1:100. 20 µl of the bacterial suspension was taken and added into 2 ml of LB(DAP+) medium containing spectinomycin; 20 µl of the bacterial suspension was taken and added into 2 ml of LB(DAP-) medium containing spectinomycin, carried out in triplicate. The bacterial strains were incubated in an air bath shaker (37°C, 220 rpm) for 72 h and the growth of the strains was observed.

Characterization under anaerobic conditions: 3 monoclones were picked and added into LB(DAP+) medium containing spectinomycin and incubated overnight in an air bath shaker (37°C, 220 rpm). The bacterial suspension after overnight incubation was placed in an anaerobic incubator and transferred at a ratio of 1:100. 20 µl of the bacterial suspension was taken and added into 2 ml of LB(DAP+) medium containing spectinomycin; 20 µl of the bacterial suspension was taken and added into 2 ml of LB(DAP-) medium containing spectinomycin, carried out in triplicate. The bacterial strains were incubated stationarily in an anaerobic incubator (37°C) for 24 h. After incubation for 24 h, the OD600 values of the samples were measured.

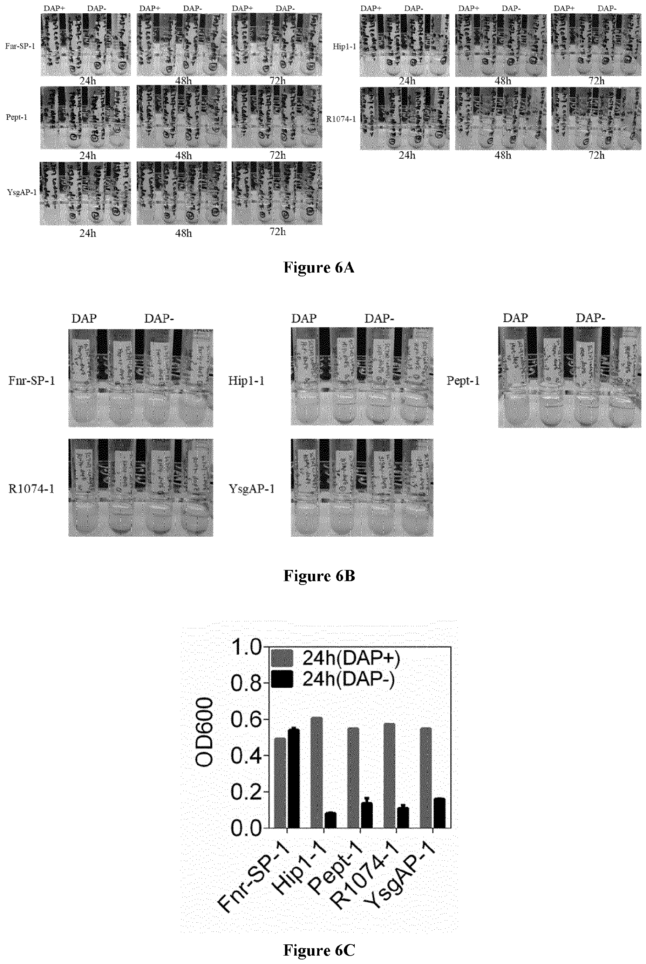

Experimental results (as shown in Figures 6A-6C):
(1) Under aerobic condition: the 5 strains were incubated in LB(DAP+) medium for 72h, and these strains could grow normally. The 5 strains were incubated in LB(DAP-) medium for 72 h, and these strains could not grow.
(2) Under anaerobic condition: 5 strains were incubated in LB(DAP+) medium and in LB(DAP-) medium for 24 h. These strains could grow in both LB(DAP+) medium and LB(DAP-) medium.

Conclusion: by means of the tests on the 5 strains under aerobic and anaerobic conditions, it showed that the facultative anaerobic strain SL7207 was successfully modified into an obligate anaerobic strain.

### Example 6: In vivo characterization of R1074-1 strain

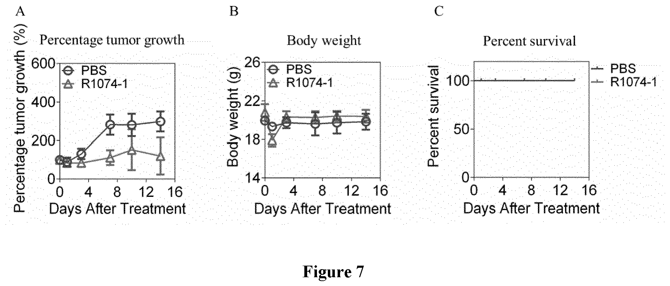

C57BL/6 mice were subcutaneously inoculated with 1×10⁶ mouse bladder cancer cells (MB49) per mouse to establish a subcutaneous tumor model of mouse bladder cancer. The experiment was divided into PBS group and R1074-1 group. The tail vein was inoculated with 1×10⁷ bacteria of the invention per mouse. The tumor volume change, mouse weight change, and percent survival of mice were detected within 14 days. Experimental results comprise (as shown in Figure 7):
(1) Tumor volume change (Figure 7A): compared with the PBS group, the R1074-1 strain group had an inhibitory effect on tumor growth during the experimental cycle.
(2) Changes in body weight of mice (Figure 7B): compared with the PBS group, the R1074-1 strain group showed no significant difference in body weight of mice during the experimental cycle
(3) Percent survival of mice: during the experimental cycle, mice in both PBS and R1074-1 strain groups survived.

Conclusion: during the experimental period, R1074-1 strain can inhibit the tumor growth in mice; meanwhile, compared with the PBS group, there is no significant difference in the body weight of the mice and all mice survived, which means that said strain is safe.

## Claims

1. A method for turning a facultative anaerobic bacterium into an obligate anaerobic bacterium by means of a circuit of hypoxically- or strictly anaerobically-induced expression of an essential gene, wherein said obligate anaerobic bacterium, when used for tumor therapy, is capable of inhibiting tumor growth and reducing tumor volume.

2. The method of claim 1, wherein said facultative anaerobic bacterium comprises a bacterium of the family Enterobacteriaceae (*Escherichia coli, Klebsiella pneumoniae, Proteus, Enterobacter, Salmonella typhi, Salmonella, Shigella,* etc.), *Staphylococcus, Streptococcus, Pneumococcus, Bacillus anthracis* and *Corynebacterium diphtheriae,* preferably said facultative anaerobic bacterium is a strain of *Salmonella sp.,* preferably a strain of facultative anaerobic *Salmonella sp.* derived from human, chicken, dog or cattle; and/or
wherein said obligate anaerobic bacterium, when being cultured *in vitro* under aerobic conditions, requires further addition of 2,6-diaminoheptanedioic acid (2,6-Diaminopimelic acid) or an analogue thereof into the culture medium; and/or
wherein said essential gene comprises *dapA* or *dapE,* and can further comprise one or more selected from the group consisting of *dapB, dapD, argD, dapF, murE, murF* or *lysA*; and/or
wherein said strictly anaerobically regulatory gene circuit consists of an anaerobically activated promoter and the essential gene; and/or
wherein said gene circuit of hypoxically- or strictly anaerobically-induced expression regulation of the essential gene is present in a chromosome or a plasmid vector.

3. The method of claim 1, wherein said anaerobically activated promoter is selected from the group consisting of Pept, Fnr-SP, Hip1, I141018, Ptet-arcA, Ptet-Fnr, R1074, Ssbp1 and YsgAP.

4. A method for treating cancer using a bacterium regulated by an anaerobic circuit, said bacterium comprising strictly hypoxic regulation of expression of an essential gene; and/or
preferably, said method further comprising: combined application with other therapy for treating cancer; and/or
preferably, wherein said combined application with other therapy for treating cancer comprises: (a) bacteriotherapy of an anaerobic strain in combination with operative therapy; (b) bacteriotherapy of an anaerobic strain in combination with radiotherapy; (c) bacteriotherapy of an anaerobic strain in combination with a chemotherapeutic which comprises alkylating agents (nimustine, carmustine, lomustine, cyclophosphamide, isocyclophosphamide, glyfosfin, etc.), antimetabolites (furtulon, doxifluridine, 6-mercaptopurine, cytarabine, fluoroguanosine, tegafur, gemcitabine, carmofur, hydroxyurea, methotrexate, tegafur-uracil, ancitabine, etc.), antitumor antibiotics (actinomycin, aclarubicin, epirubicin, mitomycin, pelomycin, bleomycin, pirarubicin, etc.), phytogenic anticarcinogens (irinotecan, harringtonine, hydroxycamptothecin, vinorelbine, paclitaxel, taxotere, topotecan, vincristine, vindesine, vinblastine, etc.), hormones (atamestane, anastrozole, aminoglutethimide, letrozole, formestane, megestrol, tamoxifen, etc.), immunosuppressants and other anticancer medicaments such as asparaginase, Carboplatin, Cisplatin, Dacarbazine, Oxaliplatin, Eloxatin, Oxaliplatin, mitoxantrone, procarbazine, etc.; (d) bacteriotherapy of an anaerobic strain in combination with biotherapy; (e) bacteriotherapy of an anaerobic strain in combination with traditional Chinese medical herbal treatment; and/or
preferably, wherein said tumor and cancer comprise leukemia (chronic leukemia, acute leukemia), osteocarcinoma, lymphoma (non-Hodgkin's lymphoma, Hodgkin's lymphoma), intestinal cancer (colon cancer, rectal cancer), liver cancer, stomach cancer, pelvic cancer (cervical cancer, malignant ovarian tumor, endometrial cancer, ovarian cancer), lung cancer, breast cancer, pancreatic cancer, bladder cancer, prostate cancer, etc.

5. A vector which is a prokaryotic cell comprising (a) a hypoxically- or strictly anaerobically-activated promoter; and (b) an essential gene under the regulation of the promoter in (a); wherein the promoter in (a) has a binding site to an anaerobically activated transcription factor.

6. The vector of claim 5, wherein said anaerobically activated promoter is selected from the group consisting of Pept, Fnr-SP, Hip1, I141018, Ptet-arcA, Ptet-Fnr, R1074, Ssbp1 and YsgAP.

7. The vector of claim 5, wherein said essential gene comprises *dapA* or *dapE,* and can further comprise one or more selected from the group consisting of *dapB, dapD, argD, dapF, murE, murF* or *lysA.*

8. The vector of claim 5, which, when being cultured *in vitro* under aerobic conditions, requires addition of 2,6-diaminoheptanedioic acid into the culture medium.

9. Use of an obligate anaerobic *Salmonella* bacterium in expressing a medicament or as a vector for carrying a medicament, said medicament being used for treating cancer.

10. The use of an obligate anaerobic *Salmonella* bacterium in expressing a medicament or as a vector carrying a medicament of claim 9, wherein said medicament comprises: (a) expressing a protein substance or a polypeptide substance having a therapeutic effect on cancer; (b) expressing RNA having a therapeutic effect on cancer; and (c) serving as a vector carrying a modified RNA medicament.
